# DEMANDE DE BREVET EUROPEEN

(11) **EP 0 567 661 A1**
(43) Date de publication de la demande: **03.11.1993**
(21) Numéro de dépôt: 92107094.2
(22) Date de dépôt: 25.04.1992
(51) Int. Cl.: C12N 9/20, C12N 11/02, C12N 9/96, A23G 1/00

(54) **Lipase modifiée, procédé de modification et utilisations**

(71) Demandeur: SOCIETE DES PRODUITS NESTLE S.A., CH-1800 Vevey (CH)
(72) Inventeur: Chmiel, Oliver, CH-1006 Lausanne (CH); Traitler, Helmut, CH-1802 Corseaux (CH)

(57) **Abrégé**

On modifie une lipase spécifique vis à vis des substrats lipidiques par réaction avec un polymère de polarité prédéterminée.

La lipase modifiée libère préférentiellement les acides gras choisis pour leur propriétés physiologiques ou d'aromatisation.

## Description

La présente invention concerne une lipase modifiée, un procédé de modification d'une lipase ainsi que les utilisations d'une lipase ainsi modifiée en nutrition et en aromatisation.

Les lipases sont des enzymes ayant la propriété d'hydrolyser ou d'estérifier les corps gras émulsionnés. Lors de ce processus, les acides gras sont libérés à partir des tri-, di- et monoglycérides. Une lipase possède une spécificité propre selon son type et son origine. Ce peut être une régiospécificité, par exemple dans le cas d'une lipase de Mucor miehei clivant sélectivement les acides gras en positions 1 et 3 du glycéride ou une spécificité pour le substrat, s'agissant par exemple d'une lipase de Geotrichum candidum ou encore une spécificité mixte résultant des deux propriétés précédentes.

On a cherché à changer les propriétés naturelles de lipases en les modifiant chimiquement pour leur conférer une meilleure solubilité dans les solvants organiques, par exemple selon EP-A-149520, en vue d'améliorer leurs activités d'hydrolyse et de synthèse dans les réactions de transestérification en milieu organique.

Nous avons trouvé de manière surprenante que l'on pouvait influencer la spécificité d'une lipase vis à vis des substrats lipidiques en la modifiant physico-chimiquement.

L'invention concerne donc une lipase spécifique vis à vis des substrats lipidiques, modifiée à partir d'une lipase native, caractérisée par le fait qu'elle présente par rapport à la lipase de départ une polarité induite par liaison avec un oligomère ou un polymère.

L'invention concerne également un procédé de modification d'une lipase en vue de changer sa spécificité vis à vis des substrats lipidiques, caractérisé par le fait que l'on fait réagir la lipase avec un oligomère ou un polymère de polarité choisie pour favoriser la libération d'acides gras de degré de saturation prédéterminé sans modifier sa spécificité vis à vis de la longueur de chaîne des acides gras libérés.

Dans le cadre de la présente invention, une lipase préférée, MAP-10 (R) de Mucor javanicus, appartient à la seconde catégorie mentionnée précédemment et possède une spécificité de substrat pour les acides gras à chaîne moyenne et longue.

Pour modifier la lipase, on la fait réagir avec un oligomère ou un polymère (dans la suite de l'exposé, on utilisera le terme polymère pour désigner aussi bien un oligomère qu'un polymère proprement dit), le cas échéant activé, de préférence de masse moléculaire 500 à 10⁶, dont la polarité va d'une absence de polarité à une valeur d'environ 1000 McReynolds. L'échelle de polarité de McReynolds (McReynolds,W. O., J. Chromatogr. Sci. 8, 685 (1970)) peut être définie sur la base des indices de rétention en chromatographie en phase gaseuse de cinq solvants de différentes polarités mesurés sur un polymère employé comme phase stationnaire dans une colonne de chromatographie en phase gazeuse, ce qui donne une évaluation de la polarité du polymère testé par comparaison avec le squalane (hydrocarbure saturé en C30 obtenu par hydrogénation du squalène) auquel on attribue par définition la valeur de polarité 0.

Les polymères mis en oeuvre peuvent être de nature chimique diverse, d'origine synthétique ou de préférence naturelle. On peut citer par exemple les diméthylpolysiloxanes et les polyéthylèneglycols greffés avec des groupes polaires ou de préférence les pectines, qui ont l'avantage de posséder déjà des groupes polaires carboxyles dans leur structure.

Dans le cas des polymères greffés, on peut réaliser le greffage qui consiste à activer le polymère en le faisant réagir avec un agent couplant monomère portant des groupes bifonctionnels polaires, par exemple dans le cas des polysiloxanes avec un glycidoxypropyltrimétoxysilane en présence d'un composé amorçant une réaction radicalaire, par exemple le dicumylperoxyde ou dans le cas d'un polyéthylèneglycol, par exemple avec le chlorure de l'acide cyanhydrique en présence d'un solvant.

On fait ensuite réagir le polymère dont la polarité a été ainsi modifiée avec l'enzyme dans des conditions de pH fortement basique dans le cas des polysiloxanes ou polyéthylèneglycols, ou à pH faiblement acide dans le cas des pectines, puis on sèche l'enzyme traitée, par exemple par lyophilisation en vue d'une utilisation ultérieure.

Nous avons constaté que la spécificité naturelle de l'enzyme ainsi transformée, par exemple dans le cas de la lipase de Mucor javanicus qui a l'état natif libère spécifiquement les acides gras à chaîne moyenne et à longue chaîne, était maintenue dans l'enzyme modifiée. Par contre, le rapport saturés/insaturés des acides gras à longue chaîne de même longueur de chaîne libérés par l'hydrolyse changeait avec la polarité du réactif constitué par le polymère par rapport à ce qui était obtenu avec l'enzyme native. Ainsi, une augmentation de la polarité du polymère avait pour conséquence un déplacement des acides gras libérés vers les insaturés. Inversément, un traitement de l'enzyme avec un polymère apolaire conduisait à la libération préférentielle d'acides gras saturés à longueur de chaîne égale.
Ce phénomène surprenant d'influence par la voie externe de la spécificité de l'enzyme peut être mis à profit dans différentes applications du domaine alimentaire.

L'invention concerne donc également un procédé d'aromatisation d'un produit alimentaire, caractérisé par le fait que l'on incorpore dans ce produit un substrat incubé avec une lipase modifiée par le procédé précédent ou que l'on traite ce produit avec une lipase modifiée par le procédé précédent.

L'invention concerne également un procédé d'amélioration des qualités nutritionelles d'un substrat lipidique, caractérisé par le fait que l'on traite un corps gras avec une lipase modifiée apolaire, c'est-à-dire de polarité augmentée de manière à en libérer dans un premier temps préférablement les acides gras saturés, puis traiter dans un deuxième temps le substrat avec une lipase modifiée polaire, ce qui conduit à introduire préférentiellement des acides gras insaturés dans le corps gras par transestérification.
Ceci a pour conséquence d'augmenter la valeur du corps gras du point de vue de la physiologie de la nutrition. L'intérêt de cette méthode est la possibilité de transformer un substrat à partir d'une lipase native de même origine, par exemple de qualité alimentaire dans un processus nécessitant plusieurs étapes.
Dans un autre ordre d'idées, on peut utiliser la propriété de modifier une enzyme spécifique libérant préférentiellement les acides gras à chaîne courte, par exemple dans la fabrication des fromages en réalisant une maturation accélérée, par exemple pour produire des arômes spécifiques. Une telle enzyme peut ainsi être modifiée dans le sens désiré, par exemple sans avoir recours à une modification par la voie génétique du microorganisme producteur de l'enzyme.
Par ailleurs, en libérant préférentiellement des acides gras insaturés à longue chaîne par action d'une enzyme modifiée de manière appropriée, on peut produire des précurseurs d'arôme puisque l'on sait que les produits de l'oxydation et de la dégradation de ces acides gras, par exemple les alcools, alcanes, aldéhydes, cétones et esters sont des arômes. On peut par exemple produire de cette manière un chocolat au lait avec un arôme de "crumb" tel que décrit dans la demande de brevet parallèle EP...de la titulaire déposée sous le titre "Procédé d'aromatisation d'un chocolat au lait".
Les enzymes peuvent bien entendu être mises en oeuvre telles quelles en solution ou en suspension dans le substrat ou sous forme immobilisées sur un support.
Les exemples ci-après illustrent l'invention. Dans ceux-ci, les parties et pourcentages sont en poids sauf indication contraire.

### Exemple 1

On dissout 2 g de diméthylpolysiloxane de masse moléculaire environ 10⁶ (SE-30,(R)), 1 g de silane monomère bifonctionnel (glycidoxypropoxysilane, A-187 (R)) et 30 mg de dicumylperoxyde dans 150 ml de chloroforme et on chauffe, puis maintient le mélange à reflux pendant 2 h. Le polymère mis en oeuvre a la polarité 44 dans l'échelle de McReynolds, c'est-à-dire qu'il n'est pratiquement pas polaire.
On évapore ensuite le solvant, puis on reprend le résidu dans 200 ml d'une solution aqueuse tampon 0,1 M de tétroxydeborate de sodium de pH 9.
On ajoute alors 0,2 g de lipase de Mucor javanicus (MAP-10,(R) de AMANO) et on maintient le mélange sous agitation pendant 1 h à la température ambiante sous azote. La réaction étant terminée, on ajuste le pH à 7 par addition d'acide chlorhydrique concentré, puis on concentre la solution par ultrafiltration et on lyophilise le concentrat.

### Exemple 2

On met 30 g de monométhoxypolyéthylèneglycol de masse moléculaire environ 1500 (PEG-1540, Carbowax (R)) en solution dans 200 ml de toluène en présence de 12 g d'un tamis moléculaire et de 12 g de carbonate de sodium. On active ensuite le polymère en le faisant réagir avec 0,8 g de chlorure de l'acide cyanhydrique pendant 2 h à 80°C sous agitation. Après refroidissement, on filtre le milieu réactionnel puis on le reprend dans l'éther de pétrole et on récupère le polymère activé. Il a une polarité de 554 dans l'échelle de McReynolds. On met en solution 0,2 g de monométhoxypolyéthylèneglycol activé et 0,2 g de lipase MAP-10 (R) dans 250 ml d'une solution aqueuse tampon 0,1 M de tétroxydeborate de sodium de pH 10, puis on agite cette solution pendant 1 h à la température ambiante sous azote.
Après réaction, on porte la solution à pH 7 par addition d'acide chlorhydrique concentré, puis on dialyse la solution et on la lyophilise.

### Exemple 3

On met en solution 3 g de dicyanoéthylpolysiloxane de masse moléculaire 10⁵-10⁶ (OV-275,(R)), 1 g de silane monomère bifonctionnel (glycidopropoxysilane, A-187 (R)) et 45 ml de dicumylperoxyde dans 200 ml d'acétone que l' on chauffe et que l'on maintient au reflux pendant 3 h. Le polymère a une polarité de 844 dans l'echelle de McReynolds. Après évaporation du solvant, on reprend le résidu dans 150 ml d'acétone, puis on y ajoute une solution de 0,2 g de lipase MAP-10 (R) dans 25 ml de tampon aqueux de pH 9 contenant 0,1 M de tétroxydeborate de sodium, puis on agite la solution pendant 1 h à la température ambiante sous azote. Après réaction, on ajuste le pH à 7 par addition d'acide chlorhydrique concentré, puis on dialyse la solution et on la lyophilise.

### Exemple 4

On mélange 1 g de pectine A de pomme de masse moléculaire 10⁵-1,5x10⁵ (méthylée à 10%) et 0,2 g de lipase MAP-10 (R) en solution aqueuse de pH 5 et l'on agite la solution pendant 1 h sous azote à la température ambiante.
Le polymère est très polaire. On sépare ensuite avec précaution par centrifugation la pectine non solubilisée, puis on ajuste le pH de la solution à 7 par addition d'une base, on concentre la solution par ultrafiltration et on lyophilise le concentrat.

### Exemple 5

On utilise les enzymes modifiées des exemples précédents pour réaliser la lipolyse du lait. Pour ce faire, on incube du lait entier avec 0,5 U/ml de lipase pendant 30 min. à 37°C. Après réaction, on reprend le substrat dans 4 fois son volume d'un mélange équivolumique de chloroforme et de méthanol sous agitation , puis on le centrifuge pendant 10 min. On recueille ensuite la phase organique que l'on applique sur des plaques de chromatographie en couche mince, puis on sépare les acides gras libres des autres composés et on forme leurs esters isopropyliques par estérification.
On analyse alors quantitativement les esters isopropyliques par chromatographie en phase gazeuse.
Les résultats de la lipolyse sont indiqués dans le tableau 1 ci-après sous forme de rapport des quantités libérées respectives des acides palmitique (C 16:0), oléique (C 18:1) et linoléique (C 18:2) sur la quantité libérée correspondante d'acide stéarique (C 18:0).

**Tableau 1**

| Polymère par polarité croissante | Rapport des quantités d'acides gras libérés | | |
|---|---|---|---|
| | C 16:0 | C 18:1 | C 18:2 |
| Exemple 1 | 2,57 | 1,59 | 0,15 |
| Exemple 2 | 3,93 | 1,45 | 0,05 |
| Exemple 3 | 3,78 | 4,43 | 2,07 |
| Exemple 4 | 3,43 | 5,08 | 0,22 |
| Lipase native | 3,15 | 2,45 | 0,14 |

On constate un changement des proportions des acides gras libérés par les enzymes modifiées en comparaison avec celles des acides gras libérés par l'enzyme native, dans le sens d'une augmentation sensible des acides gras insaturés de longueur de chaîne identique.

### Exemple 6

Les effets de la lipolyse par l'action de la lipase MAP-10 (R) native (3) et de la lipase modifiée selon l'exemple 4 (2) ont été testés dans les mêmes conditions d'incubation sur un fromage frais instantané reconstitué à partir de 70 g de poudre dans 210 ml d'eau par comparaison avec le fromage standard (1) au moyen de tests de dégustation à l'aveugle.
La lipolyse a été effectuée par addition de 30 mg d'enzyme solubilisée dans 5 ml d'eau à 70 g de fromage reconstitué dans 205 ml d'eau et incubation pendant 30 min à la température ambiante. Les fromages ont été ensuite refroidis à env. 8-10°C.
Le fromage (2) a montré un goût de fromage semblable à celui de (1) en plus intense, mais sans apparition des mauvaises odeurs ni de goût de vieux obtenus avec (3).
Les notes hédoniques moyennes étaient 6 pour (1), 7,6 pour (2) et 8,8 pour (3) (0 = meilleure note, 10 = moins bonne note).

### Exemple 7

Dans le but de produire des sauces au fromage au goût de Cheddar, on a incorporé un concentrat de lait entier ultrafiltré lipolysé soit par la lipase native MAP-10 (R) (b), soit par la lipase modifiée selon l'exemple 4 (c) en remplacement partiel du Cheddar dans la formule standard (a). Le lait ultrafiltré a été incubé avec les lipases à 50°C pendant 24 h.
La composition des sauces est indiquée au tableau 2 ci-après.

**Tableau 2**

| Ingrédient | Concentration (%) | | |
|---|---|---|---|
| Eau | 68,05 | 68 | 69,35 |
| Cheddar | 10 | 6 | 6 |
| Concentrat lipolysé (b) | -- | 4 | -- |
| Concentrat lipolysé (c) | -- | -- | 4 |
| Arôme de Cheddar | 0,8 | 0,8 | -- |
| Amidons de maïs | 13,8 | 13,8 | 13,8 |
| Huile de soja | 3,9 | 3,9 | 3,9 |
| Sels, colorants, épaississants | complément à 100 | | |
| Note hédonique (0 = meilleure, 10 = moins bonne) | 2,8 | 1,8 | 1,4 |

Par rapport à la sauce standard (a), celle contenant le concentrat lipolysé avec la lipase native (b) avait une odeur et un goût de Cheddar prononcés et celle contenant le concentrat lipolysé avec la lipase modifiée (c) avait une note butyrique et un goût de noix légers et agréables.

## Revendications

1. Lipase spécifique vis à vis des substrats lipidiques, modifiée à partir d'une lipase native, caractérisée par le fait qu'elle présente par rapport à la lipase de départ une polarité induite par liaison avec un oligomère ou un polymère.

2. Lipase selon la revendication 1, caractérisée par le fait qu'elle présente une spécificité de substrat pour les acides gras à chaîne moyenne et longue et que sa polarité est modifiée par réaction avec un oligomère ou un polymère polaire.

3. Lipase selon la revendication 2, caractérisée par le fait que sa polarité est modifiée par liaison avec une pectine.

4. Procédé de modification d'une lipase en vue de changer sa spécificité vis à vis des substrats lipidiques, caractérisé par le fait que l'on fait réagir la lipase avec un oligomère ou un polymère de polarité choisie pour favoriser la libération d'acides gras de degré de saturation prédéterminé sans modifier sa spécificité vis à vis de la longueur de chaîne des aciders gras libérés.

5. Procédé selon la revendication 4, caractérisé par le fait que l'on fait réagir une lipase présentant une spécificité de substrat pour les acides gras à chaîne moyenne et longue avec un oligomère ou un polymère polaire.

6. Procédé selon la revendication 5, caractérisé par le fait que l'on fait réagir la lipase avec une pectine.

7. Procédé d'aromatisation d'un produit alimentaire, caractérisé par le fait que l'on incorpore dans ce produit un substrat incubé avec une lipase modifiée par le procédé selon l'une des revendications 1 à 6 ou que l'on traite ce produit avec une lipase modifiée par le procédé selon l'une des revendications 1 à 6.

8. Procédé d'amélioration des qualités nutritionelles d'un substrat lipidique, caractérisé par le fait que l'on traite un corps gras avec une lipase modifiée apolaire, c'est-à-dire de polarité augmentée de manière à en libérer dans un premier temps préférablement les acides gras saturés, puis traiter dans un deuxième temps le substrat avec une lipase modifiée polaire, ce qui conduit à introduire préférentiellement des acides gras insaturés dans le corps gras par transestérification.
